# EUROPEAN PATENT SPECIFICATION

(11) **EP 2 984 489 B1**
(45) Date of publication and mention of the grant of the patent: **06.11.2019**
(21) Application number: 14782156.5
(22) Date of filing: 04.04.2014
(51) Int. Cl.: G01N 33/82

(54) **ASSAYS FOR ANALYTE HOMOLOGS**
ASSAYS FÜR ANALYTHOMOLOGE
ESSAIS POUR DES HOMOLOGUES D'ANALYTE

(30) Priority: 12.04.2013 US 201313861422
(43) Date of publication of application: 17.02.2016
(73) Proprietor: Siemens Healthcare Diagnostics Inc., Tarrytown, NY 10591 (US)
(72) Inventor: WEI, Tie Q., Wilmington, Delaware 19808 (US); BEDZYK, William, Odessa, Delaware 19730 (US); GARCIA, Elsa, New Castle, Delaware 19720 (US)
(74) Representative: Schweitzer, Klaus
(86) International application number: PCT/US2014/032967
(87) International publication number: WO 2014/168826

(56) References cited:
- WO-A1-2014/134139
- US-A1- 2010 140 086
- US-A1- 2012 190 046
- US-A1- 2013 059 825
- US-A1- 2014 242 615
- Anonymous: "PRODUCT DATA SHEET Sheep monoclonal antibodies (SMAs) to: 25-hydroxy vitamin D (25-OH D)", , 1 October 2010 (2010-10-01), XP055314432, Retrieved from the Internet: URL:https://web.archive.org/web/2015031317 5041/http://www.bioventix.com/PDSvitDNov20 10.pdf [retrieved on 2016-10-27]
- P GLENDENNING ET AL: "Current assays overestimate 25-hydroxyvitamin D3 and underestimate 25-hydroxyvitamin D2 compared with HPLC: need for assay-specific decision limits and metabolite-specific assays Introduction", ANNALS OF CLINICAL BIOCHEMISTRY, vol. 43, 1 January 2006 (2006-01-01), pages 23-30, XP055314370, DOI: 10.1258/000456306775141650
- HUGHES ET AL.: 'Radioligand Receptor Assay for 25-Hydroxyvitamin D2/D3 and la, 25- Dihydroxyvitamin D2/D3' THE JOURNAL OF CLINICAL INVESTIGATION vol. 58, 1976, pages 61 - 70, XP055292099

## Description

This application claims benefit of non-provisional application US Serial No. 13/861,422, filed April 12, 2013.

### BACKGROUND

This invention relates to compositions, methods and kits for accurately determining the total amount of analyte homologs in a sample suspected of containing the analyte homologs.

Many small molecule compounds or haptens such as, for example, drugs and vitamins, exist in homologic forms, each of which contribute to the overall biological function of the compound either directly or through the intermediacy of one or more of their metabolic products. In order to obtain an accurate measurement of the total amount of active analyte, it is important to measure substantially equimolar amounts of the homologic forms because, where the amounts of the homologic forms are unequal, the signal obtained in an assay is distorted and does not represent the total amount of the homologic forms. Some of the issues surrounding the measurement of substantially equimolar amounts of analyte homologs include, for example, the difficulty of obtaining an antibody that binds equally to the analyte homologs and the unequal release of analyte homologs that are bound by endogenous binding substances.

There is a need for reagents and methods for accurate and sensitive determinations of total amounts of an analyte that exists in homologic forms, all of which are active, in samples suspected of containing such analytes. For example, there is a need for reagents and methods for accurate and sensitive determinations of concentrations of homologic forms of vitamin D.

### SUMMARY

Described herein are methods of adjusting a contribution of one of two analyte homologs to an amount of signal obtained in an assay for determining a total amount of the two analyte homologs in a sample. In the method, a combination is provided in an assay medium. The combination comprises a
sample suspected of containing the two analyte homologs, reagents for conducting an assay for determining the total amount of the two analyte homologs in the sample, wherein the reagents comprises at least one assay receptor that binds to the two analyte homologs, and a non-assay receptor that has a greater binding affinity for whichever of the two analyte homologs whose contribution to the amount of signal is to be adjusted. An amount of the non-assay receptor is sufficient to achieve an adjustment of the contribution of the analyte homolog to the signal. The assay for determining the total amount of the two analyte homologs is conducted.

Further, described herein are methods of determining in a sample an amount of a first analyte homolog and a second analyte homolog. In the method, a combination is provided in an assay medium. The combination comprises a sample suspected of containing the first analyte homolog and the second analyte homolog, reagents for conducting an assay for the first analyte homolog and the second analyte homolog, and a non-assay receptor that has a greater binding affinity for whichever of the first analyte homolog or the second analyte homolog whose contribution to the amount of signal is to be adjusted. The reagents for conducting an assay comprise an assay antibody capable of binding to each of the first analyte homolog and the second analyte homolog to form complexes and a member of a signal producing system that produces a signal in relation to the amount of the first analyte homolog and the second analyte homolog in the complexes. An amount of the non-assay receptor is sufficient to achieve an adjustment of the contribution of the first analyte homolog or the second analyte homolog to the signal. The assay medium is incubated under conditions for forming the complexes. An amount of signal from the complexes is determined and related to the total amount of the first analyte homolog and the second analyte homolog in the sample.

Some examples in accordance with the principles described herein are directed to methods of determining in a sample a total amount of 25-hydroxy vitamin D₂ and 25-hydroxy vitamin D₃ according to claim 1. A combination is provided in an assay medium. The combination comprises a sample suspected of containing 25-hydroxy vitamin D₂ and 25-hydroxy vitamin D₃, reagents for conducting an assay for 25-hydroxy vitamin D₂ and 25-hydroxy vitamin D₃, and a non-assay antibody that has a greater binding affinity for 25-hydroxy vitamin D₂. The reagents for conducting the assay comprise an assay antibody capable of binding to 25-hydroxy vitamin D₂ and 25-hydroxy vitamin D₃ to form complexes and a member of a signal producing system that produces a signal in relation to the amount of 25-hydroxy vitamin D₂ and 25-hydroxy vitamin D₃ complexes. An amount of the non-assay antibody is sufficient to achieve an adjustment of the contribution of 25-hydroxy vitamin D₂ to the amount of signal. The assay medium is incubated under conditions for forming the complexes. The amount of signal from the complexes is determined and is related to the total amount of 25-hydroxy vitamin D₂ and 25-hydroxy vitamin D₃ in the sample.

### BRIEF DESCRIPTION OF DRAWINGS

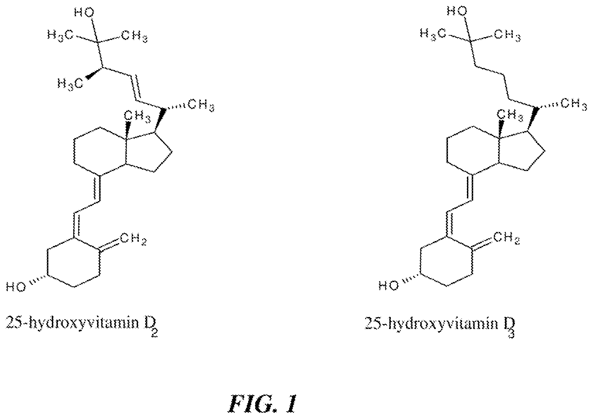

Fig. 1 is a depiction of the chemical formulas for the homologic forms of vitamin D, namely, 25-hydroxyvitamin D₂ and 25-hydroxyvitamin D₃.

### DETAILED DESCRIPTION OF SPECIFIC EMBODIMENTS

### General Discussion

Herein, methods are described which are directed to adjusting a contribution of two or more analyte homologs to an amount of signal obtained in an assay for determining a total amount of the analyte homologs in a sample. There are a number of reasons why the contribution of an analyte homolog to the signal obtained in an assay may result in an inaccurate determination of the total amount of the analyte homologs in a sample. These reasons include, but are not limited to, an assay antibody not binding equally to the different analyte homologs present in a sample and/or the fact that one of the analyte homologs may be displaced from endogenous binding substances by a displacing agent in an amount greater than that for another analyte homolog. Both of the above situations give rise to the generation of unequal amounts of signal generated where the contribution of one of the analyte homologs to the amount of signal renders the total signal amount erroneously reflective of the total amount of the analyte homologs in the original sample. For example, by way of illustration and not limitation, there are two potential factors that could contribute to higher assay signal for 25-hydroxy vitamin D₂. The first factor is that 25-hydroxy vitamin D₂ binds to the vitamin D binding proteins less strongly than 25-hydroxy vitamin D₃ so 25-hydroxy vitamin D₂ is more easily released from the vitamin D binding proteins and gives out a higher concentration of free 25-hydroxy vitamin D₂ molecules that are accessible by an assay antibody. The second factor is that in some instances the assay antibody has a greater affinity to the 25-hydroxy vitamin D₂ analog than to 25-hydroxy vitamin D₃. The two factors working together can result in much higher signal from 25-hydroxy vitamin D₂ than from 25-hydroxy vitamin D₃, which is the root cause of non-equimolarity.

Performance of a particular assay format at the low end of a medical decision range may be monitored by monitoring a difference in an amount of signal obtained for calibrators spanning the suspected concentration range of interest of an analyte. A large difference or separation between the signal for calibrators such as, for example, calibrator L1 and calibrator L2 or calibrator L2 and calibrator L3, or calibrator L1 and calibrator L4, for example, is desired. In some examples, five calibrators may be employed, arbitrarily named L1-L5. Signal to noise ratio may be evaluated by determining an amount of signal using a calibrator that contains no analyte, arbitrarily designated calibrator L1 (background), and the amount of signal obtained for a calibrator containing a first known amount of analyte above zero, arbitrarily designated calibrator L2. This evaluation may also include determining an amount of signal using calibrator L1 and the amount of signal for a calibrator containing a second known amount of analyte above zero, arbitrarily designated L3. Such an evaluation would also include such determination using calibrators L4 and L5, wherein each of the calibrators contains known increasing amounts of analyte. Depending on the assay format, the difference in signal may be an increase in signal or a decrease in signal. For example, for a competitive assay, in most instances there will be a decrease in signal related to the concentration of analyte; and, for a sandwich assay, in most instances there will be an increase in signal related to the concentration of analyte.

The amounts of signal obtained from assays on the calibrators formed using known concentrations of the analyte are plotted against the concentration of analyte in each calibrator to form a calibration curve or standard curve. Results obtained in assays on samples containing unknown amounts of signal are compared to the calibration curve to ascertain the amount of analyte in the unknown sample based on the amount of signal obtained in an assay for the analyte.

Methods in accordance with the principles described herein measure total amounts of two or more analyte homologs where the analyte homologs or their respective metabolic products are potent. The phrase "analyte homologs" or "homologic forms of an analyte" refers to forms of an analyte that are related by structure and function and that differ from one another by one or more chemical moieties such as, for example, methylene groups, methyl groups, hydrogens, keto groups and epimers, for example. The term "potent" refers to the degree of an activity of an analyte with respect to a particular function, which may be, for example, a biological function such as, e.g., bone metabolism. For example, biological activity of a substance relates to the ability of the substance to enhance or suppress a biological function such as, for example, maintaining appropriate levels of minerals and salts in a subject, cellular function. Vitamin D, by way of illustration and not limitation, maintains appropriate levels of calcium and phosphate in a subject, which relate to calcium homeostasis and bone metabolism.

Accurately assessing the total amount of an analyte that has two or more homologic forms in biological samples is important particularly where the two or more homologic forms are potent. The presence of the analyte may be indicative of, for example, a disease state, a deficiency, a gene mutation, or a metabolic pattern related to pharmacogenetics. For example, measuring vitamin D levels in biological samples is important since vitamin D deficiency is related to a number of disorders in mammals. In infants, for example, vitamin D measurements that are inaccurate lead to inaccurate assessment of vitamin D levels in the infant, which in turn can lead to a lack of proper supplementation. It is important to measure the total amount of the active forms of vitamin D so that an infant can receive proper vitamin D therapy, if necessary.

The term "vitamin D" refers to a group of fat-soluble secosteroids. In humans, vitamin D is unique because it can be ingested as cholecalciferol (vitamin D₃) or ergocalciferol (vitamin D₂) and because the body can also synthesize it (from cholesterol) when sun exposure is adequate. Because of this latter property, vitamin D is considered by some to be a non-essential dietary vitamin although most consider it an essential nutrient. Vitamin D has an important physiological role in the positive regulation of calcium ion homeostasis. Vitamin D₃ is the form of the vitamin synthesized by animals. It is also a common supplement added to milk products and certain food products as is vitamin D₂. The structures for 25-hydroxyvitamin D₃ and 25-hydroxyvitamin D₂ are set forth in Fig. 1.

Both dietary and intrinsically synthesized vitamin D₃ and vitamin D₂ must undergo metabolic activation to generate bioactive metabolites. In humans, the initial step of vitamin D₃ activation occurs primarily in the liver and involves hydroxylation to form the intermediate metabolite 25-hydroxycholecalciferol (also referred to as calcidiol, calcifediol, 25-hydroxycholecalciferol, or 25-hydroxyvitamin D₃. Calcidiol is the major form of Vitamin D₃ in the circulatory system. Vitamin D₂ also undergoes similar metabolic activation to 25-hydroxyvitamin D₂. Collectively these compounds are called 25-hydroxyvitamin D (abbreviated 25(OH)D) and they are the major metabolites that are measured in serum to determine vitamin D status; 25(OH)D are pre-hormones that need to be converted into 1,25(OH)D to exert biological functions.

Methods in accordance with the principles described herein provide for adjusting a contribution of one of two analyte homologs to an amount of signal obtained in an assay for determining a total amount of the two analyte homologs in a sample particularly where one receptor or specific binding pair member such as an antibody is employed that binds to all of the analyte homologs in a sample. In some examples, it is important to adjust an amount of signal from one homolog over that of another homolog in order to have the total amount of signal measured be reflective of substantially equimolar amounts of the analyte homologs in a sample. As mentioned above, some of the issues surrounding the measurement of substantially equimolar amounts of analyte homologs include, for example, the difficulty of obtaining receptor such as an antibody that binds equally to the analyte homologs and the unequal release of analyte homologs that are bound by endogenous binding substances. The phrase "substantially equimolar amounts" means that the amounts of the analyte homologs in the assay medium are equivalent or differ by no more than, for example, 10%, or 9%, or 8%, or 7%, or 6%, or 5%, or 4%, or 3%, or 2%, or 1%, or 0% from one another.

Ideally, for an assay for two or more analyte homologs, one receptor such as an antibody is employed that binds equally to the two or more analyte homologs. However, in many instances such a receptor is difficult to obtain and a receptor that binds unequally to the two or more analyte homologs must be used. In situations where a receptor that binds unequally to the analyte homologs is employed, the assay may yield an inaccurate result for the total concentration of the analyte homologs in a sample. In such situations the calibrators are typically formed using only one of the analyte homologs. If a receptor used in the assay binds more vigorously to one of the analyte homologs, more signal is generated from that analyte homolog and the overall amount of signal is increased. When this amount of overall signal is compared to the calibration curve, the corresponding total amount of analyte homologs obtained from the calibration curve will be higher than the actual total amount of analyte homologs in a sample. Methods in accordance with the principles described herein provide means for adjusting the amount of signal obtained so that the signal can be accurately related to the actual total amount of the analyte homologs in a sample.

As mentioned above, another reason for inaccurate signal obtained in an assay for analyte homologs is the unequal release of analyte homologs that are bound by endogenous binding substances. Many analyte homologs are bound by endogenous binding substances. Such endogenous binding substances are those that are present in a sample taken from its source. The nature of the endogenous binding substances is dependent on one or more of the nature of the sample, the nature of the source of the sample, the nature of the analyte, and the nature of a molecular complex comprising the analyte, for example.

Analyte homologs bound by endogenous binding substances may be released from such substances by a displacing agent as discussed herein. Endogenous binding substances include both endogenous specific binding substances and endogenous non-specific binding substances. Specific binding substances are substances that have an area on a surface or in a cavity, which specifically binds to and is thereby defined as complementary with, a particular spatial and polar organization of an analyte or vice versa. Specific binding is distinguished from non-specific binding because specific binding involves the specific recognition of one of two different molecules for the other compared to substantially less recognition of other molecules. Non-specific binding substances are substances that bind to an analyte, in general, by means of non-covalent binding between molecules that is relatively independent of specific surface structures. Endogenous binding substances for an analyte include, but are not limited to, proteins that specifically bind to an analyte such as, for example, anti-analyte antibodies and receptors, and binding proteins that bind non-specifically to an analyte. In a particular example, by way of illustration and not limitation, the analyte is vitamin D and the endogenous binding substances are vitamin D binding proteins.

In instances that involve endogenous binding substances, a displacing agent may be employed to release the analyte from the endogenous binding substances. One problem with the use of displacing agents is that in many instances the displacing agent does not displace analyte homologs in equal amounts from endogenous binding substances. Rather, one analyte homolog may be displaced in greater amounts relative to the displacement of another homolog of that same analyte. This phenomenon contributes to the problem of not having substantially equimolar amounts of signal obtained in an assay even where a receptor for the analyte homologs employed in the assay binds substantially equally to the analyte homologs for detection in an assay. Methods in accordance with the principles described herein provide means for adjusting the amount of signal obtained so that the signal can be accurately related to the actual total amount of the analyte homologs in a sample.

As mentioned above, measurements of the analyte homologs may be carried out on samples that have been treated with a displacing agent. The amount of displacing agent that is added to the sample is that which is sufficient to displace substantially all of the analyte homologs from the endogenous binding substances. The phrase "displace substantially all of the analyte homologs from the endogenous binding substances" means that the analyte homologs are at least 80%, or at least 90%, or at least 95%, or at least 99%, or at least 99.5%, or at least 99.9% or is 100% displaced from endogenous binding substances and available for detection during an assay.

After addition of a displacing agent, the sample is incubated for a period of time under conditions to displace substantially all of the analyte homologs from endogenous binding substances. The length and conditions of the incubation are dependent on one or more of the nature of the displacing agent, the nature of the analyte homologs, and the suspected concentration of the analyte homologs, for example. In some embodiments incubation temperatures for this step may be 5°C to 99°C, or 15°C to 70°C, or 20°C to 45°C, for example. The time period for the incubation is 0.2 seconds to 24 hours, or 1 second to 6 hours, or 2 seconds to 1 hour, or 1 to 15 minutes, for example. The incubation may be carried out in a medium that, for convenience, may be an assay medium as discussed herein, but need not be.

In assay methods in accordance with the principles described herein, a combination is provided in an assay medium. The combination comprises a sample suspected of containing two analyte homologs, reagents for conducting an assay for determining the total amount of the two analyte homologs in the sample, wherein the reagents comprises at least one assay receptor that binds to the two analyte homologs, and a non-assay receptor that has a greater binding affinity for whichever of the two analyte homologs whose contribution to the amount of signal is to be adjusted. An amount of the non-assay receptor is sufficient to achieve a predetermined amount of an adjustment of the contribution of the analyte homolog to the signal. The assay for determining the total amount of the two analyte homologs is conducted.

The non-assay receptor is a substance that binds preferentially to one analyte homolog over another analyte homolog and that is not involved in forming a complex related to the presence or amount of the analyte homologs in a sample. The non-assay receptor may be, by way of illustration and not limitation, an antibody, a molecular imprinting polymer, anti-sense RNA or anti-sense DNA, for example.

The assay antibody exhibits sufficient assay binding affinity for the analyte homologs suspected of being in the sample. The non-assay antibody binds to the excessive analyte homolog with a binding affinity that is greater than the binding affinity for the non-excessive analyte homolog. In some examples the non-assay antibody exhibits sufficient binding affinity for the excessive analyte homolog but exhibits insufficient binding affinity or substantially no binding affinity for the non-excessive analyte homolog.

The phrase "assay binding affinity" refers to the strength with which an assay antibody binds to corresponding analyte homologs to produce a complex of assay antibody bound to the analyte homologs. An antibody may be monoclonal or polyclonal. Antibodies may include a complete immunoglobulin or fragment thereof, which immunoglobulins include the various classes and isotypes, such as IgA, IgD, IgE, IgG1, IgG2a, IgG2b and IgG3, IgM, etc. Fragments thereof may include Fab, Fv and F(ab')₂, Fab', and the like. In addition, aggregates, polymers, and conjugates of immunoglobulins or their fragments can be used where appropriate so long as binding affinity for a particular molecule is maintained.

Monoclonal antibodies can be prepared by techniques that are well known in the art such as preparing continuous hybrid cell lines and collecting the secreted protein (somatic cell hybridization techniques). Monoclonal antibodies may be produced according to the standard techniques of Köhler and Milstein, Nature 265:495-497, 1975. Reviews of monoclonal antibody techniques are found in Lymphocyte Hybridomas, ed. Melchers, et al. Springer-Verlag (New York 1978), Nature 266: 495 (1977), Science 208: 692 (1980), and Methods of Enzymology 73 (Part B): 3-46 (1981).

In another approach for the preparation of antibodies, the sequence coding for antibody binding sites can be excised from the chromosome DNA and inserted into a cloning vector, which can be expressed in bacteria to produce recombinant proteins having the corresponding antibody binding sites. This approach involves cloning and expressing nucleotide sequences or mutagenized versions thereof coding at least for the amino acid sequences required for specific binding of natural antibodies.

In one approach for the production of monoclonal antibodies, a first step includes immunization of an antibody-producing animal such as a mouse, a rat, a goat, a sheep, or a cow with the antigen, for example, with an immunogen. Immunization can be performed with or without an adjuvant such as complete Freund's adjuvant or other adjuvants such as monophosphoryl lipid A and synthetic trehalose dicorynomycolate adjuvant. A next step includes isolating spleen cells from the antibody-producing animal and fusing the antibody-producing spleen cells with an appropriate fusion partner, typically a myeloma cell, such as by the use of polyethylene glycol or other techniques. Typically, the myeloma cells used are those that grow normally in hypoxanthine-thymidine (HT) medium but cannot grow in hypoxanthine-aminopterin-thymidine (HAT) medium, used for selection of the fused cells. A next step includes selection of the fused cells, typically by selection in HAT medium. A next step includes screening the cloned hybrids for appropriate antibody production using immunoassays such as enzyme-linked immunosorbent assay (ELISA) or other immunoassays appropriate for screening.

An assay antibody with the requisite binding affinity for analyte homologs as set forth above may be selected by well-known screening methodologies, which include, by way of illustration and not limitation, ELISA, dot blots, Western analysis, and Surface Plasmon Resonance, for example.

As mentioned above, the function of the non-assay receptor is to provide for adjusting a contribution of one of two analyte homologs to an amount of signal obtained in an assay for determining a total amount of the two analyte homologs in a sample. In some examples in accordance with the principles described herein, the non-assay receptor provides for substantially equimolar amounts of signal in the assay. Non-assay receptors such as non-assay antibodies are chosen for this purpose. For example, a non-assay antibody is selected for its ability (its binding affinity) to bind one analyte homolog over another analyte homolog. A non-assay antibody should exhibit a preferential binding affinity for an analyte homolog that is known to be in excess in the assay medium as described above.

Accordingly, a non-assay antibody is prepared and selected by means of an appropriate screening method such that the non-assay antibody exhibits sufficient binding affinity for an analyte homolog over another analyte homolog such that signal obtained in an assay for the analyte homologs is adjusted for one or both of the inequality of the binding affinity of an assay receptor for the analyte homologs and the unequal release of one of the analyte homologs from endogenous binding substances. For purposes of this description, an analyte homolog for which an assay receptor has a greater binding affinity or which is released in greater amounts from endogenous binding substances is referred to herein as the "excessive analyte homolog" and the other analyte homolog is referred to herein as the "non-excessive analyte homolog.

In some examples in accordance with the principles described herein, the non-assay receptor is a non-assay antibody. A non-assay antibody with the requisite binding affinity for an analyte homolog as set forth above may be selected by well-known screening methodologies, which include, by way of illustration and not limitation, ELISA, dot blots, Western analysis, and Surface Plasmon Resonance, for example.

The phrase "preferential binding affinity" means that the binding affinity of the non-assay antibody for the excessive analyte homolog is greater than the binding affinity of the non-assay antibody for the non-excessive analyte homolog. In some examples in accordance with the principles described herein, the non-assay antibody has a binding affinity for the excessive analyte homolog that is greater that its binding affinity for the non-excessive analyte homolog by a factor, for example, of 10, or 10², or 10³, or 10⁴, or 10⁵. For example, if the binding affinity of the non-assay antibody for the excessive analyte homolog is 10⁹ liters/mole, the binding affinity of the non-assay antibody for the non-excessive analyte homolog may be less that 10⁷ liters/mole, or less than 10⁶ liters/mole, for example. In some examples in accordance with the principles described herein, the non-assay antibody exhibits substantially no binding affinity for the non-excessive analyte homolog. The phrase "exhibits substantially no binding affinity" means that substantially no detectable complexes are formed between the non-assay antibody and the non-excessive analyte homolog.

In some examples in accordance with the principles described herein, the binding affinity of the non-assay antibody for the excessive analyte homolog is at least 10⁷ liters/mole, or at least 10⁸ liters/mole, or at least 10⁹ liters/mole, or at least 10¹⁰ liters/mole, or at least 10¹¹ liters/mole, or at least 10¹² liters/mole, or at least 10¹³ liters/mole, or at least 10¹⁴ liters/mole, for example. In some examples in accordance with the principles described herein, the binding affinity of the non-assay antibody for the excessive analyte homolog is 10⁷ to 10¹⁴ liters/mole, or 10⁷ to 10¹¹ liters/mole, or 10⁷ to 10¹² liters/mole, or 10⁸ to 10¹⁴ liters/mole, or 10⁸ to 10¹¹ liters/mole, or 10⁸ to 10¹² liters/mole, for example.

In some examples in accordance with the principles described herein, the binding affinity of the non-assay antibody for the non-excessive analyte homolog is less than 10⁷ liters/mole, or less than 10⁶ liters/mole, or less than 10⁵ liters/mole, or less than 10⁴ liters/mole, or less than 10³ liters/mole, or less than 10² liters/mole, or less than 10 liters/mole, for example. In some examples in accordance with the principles described herein, the binding affinity of the non-assay antibody for the non-excessive analyte homolog is 10 to 10⁶ liters/mole, or 10 to 10⁵ liters/mole, or 10 to 10⁴ liters/mole, or 10 to 10³ liters/mole, or 10 to 10² liters/mole, or 10² to 10⁶ liters/mole, for example.

An amount of the non-assay receptor employed is sufficient to achieve an adjustment of the contribution of the excessive analyte homolog to the signal obtained in an assay for the total amount of the analyte homologs. In some examples, an amount of the non-assay receptor in the assay medium is that sufficient to achieve substantially equal amounts of signal for the excessive analyte homolog and the non-excessive analyte homolog in the assay medium. The amount of the non-assay receptor is dependent on a number of factors that include, by way of illustration and not limitation, the nature and binding affinity of the non-assay receptor for the different analyte homologs, the nature and binding affinity of the assay receptor for the different analyte homologs, the amount of the excessive analyte homolog released by a displacing agent over the amount of the non-excessive analyte homolog released, the nature of the analyte homologs, the nature of the assay, the nature of the assay reagents, and the nature of the clinical samples, for example. In some examples the amount of the non-assay receptor employed is determined empirically and is based on one or more of the above factors. In some examples in accordance with the principles described herein, the amount of the non-assay receptor is 10 µg/mL to 75 µg/mL, or 10 µg/mL to 50 µg/mL, or 10 µg/mL to 25 µg/mL, for example.

The treatment of the medium comprising the analyte homologs may be carried out as a separate step or in conjunction with an assay for the analyte homologs. Where the amount of the excessive analyte homolog results from treatment of the sample with a releasing agent as discussed herein, the treatment of the medium with a non-assay receptor is carried out subsequent to the treatment with a releasing agent. Where the amount of excessive analyte homolog results from the inequality in the assay binding affinity of an assay antibody for the analyte homologs, treatment of the medium with a non-assay receptor is carried out during the assay for the analyte homologs.

The conditions for treating the medium comprising the analyte homologs in accordance with the principles described herein may be the same as those employed in an assay for the analyte homologs. In some examples, the treatment is carried out in an aqueous buffered medium at a moderate pH, generally that which provides optimum binding of the non-assay receptor to the excessive analyte homolog. The aqueous medium may be solely water or may include from 0.1 to 40 volume percent of a cosolvent. The pH for the medium will be in the range of 4 to 11, or in the range of 5 to 10, or in the range of 6.5 to 9.5, for example. The pH will usually be a compromise between optimum binding of the binding members of any specific binding pairs, the pH optimum for other reagents of the assay such as members of the signal producing system, and so forth. Various buffers may be used to achieve the desired pH and maintain the pH during the assay. Illustrative buffers include, by way of illustration and not limitation, borate, phosphate, carbonate, TRIS, barbital, PIPES, HEPES, MES, ACES, MOPS, and BICINE, for example. The particular buffer employed is not critical, but in an individual assay one or another buffer may be preferred. Various ancillary materials may be employed in the treatment with a non-assay receptor. For example, in addition to buffers the medium may comprise stabilizers for the medium and for the reagents employed.

One or more incubation periods may be applied to the medium at one or more intervals including any intervals between additions of various reagents employed in a treatment with a non-assay receptor. The medium is usually incubated at a temperature and for a time sufficient for binding of the binding of a non-assay receptor to an excessive analyte homolog. Moderate temperatures are normally employed for carrying out the method and usually constant temperature, preferably, room temperature, during the period of the measurement. In some examples, incubation temperatures range from 5° to 99°C, or from 15°C to 70°C, or from 20°C to 45°C, for example. The time period for the incubation, in some examples, is 0.2 seconds to 24 hours, or 1 second to 6 hours, or 2 seconds to 1 hour, or 1 minute to 15 minutes, for example. The time period depends on a number of factors that include, but are not limited to, the temperature of the medium and binding affinity of the non-assay receptor for the excessive analyte homolog and for the non-excessive analyte homolog, where appropriate, for example.

An assay for the first analyte homolog and the second analyte homolog is then carried out to determine an amount of the first analyte homolog and the second analyte homolog in a sample. The reagents for conducting an assay comprise an assay receptor such as, for example, an assay antibody capable of binding to each of the analyte homologs to form complexes and a member of a signal producing system that produces a signal in relation to the amount of the analyte homologs in the complexes. An amount of the non-assay receptor is sufficient to achieve an adjustment of the contribution of the first analyte homolog or the second analyte homolog to the signal. The assay medium is incubated under conditions for forming the complexes. An amount of signal from the complexes is determined and related to the total amount of the analyte homologs in the sample. The assay may be chosen from any number of assays as discussed in more detail below.

### General Description of Assays

The following discussion is by way of illustration and not limitation. Any appropriate assay that utilizes an antibody (immunoassay) may be employed in the determinations involved in accordance with the principles described herein. The assays can be performed either without separation (homogeneous) or with separation (heterogeneous) of any of the assay components or products. Heterogeneous assays usually involve one or more separation steps and can be competitive or non-competitive. The assays may be manual or automated.

The sample to be analyzed is one that is suspected of containing two or more analyte homologs. The samples may be biological samples or non-biological samples. Biological samples may be from a mammalian subject or a non-mammalian subject. Mammalian subjects may be, e.g., humans or other animal species. Biological samples include biological fluids such as whole blood, serum, plasma, sputum, lymphatic fluid, semen, vaginal mucus, feces, urine, spinal fluid, saliva, stool, cerebral spinal fluid, tears, mucus, and the like; biological tissue such as hair, skin, sections or excised tissues from organs or other body parts; and so forth. In many instances, the sample is whole blood, plasma or serum. Non-biological samples including, but not limited to, waste streams, for example, may also be analyzed using compounds in accordance with the principles described herein.

The sample can be prepared in any convenient medium, which may be, for example, an assay medium, which is discussed more fully below. In some instances a pretreatment may be applied to the sample such as, for example, to release the analyte homologs from endogenous binding substances or to lyse blood cells. In some examples, such pretreatment is performed in a medium that does not interfere subsequently with an assay.

In many embodiments immunoassays involve labeled reagents. Immunoassays that involve labeled reagents include chemiluminescence immunoassays, enzyme immunoassays, fluorescence polarization immunoassays, radioimmunoassays, inhibition assay, induced luminescence assays, and fluorescent oxygen channeling assays, for example.

One general group of immunoassays includes immunoassays using a limited concentration of one of the assay reagents. Another group of immunoassays involves the use of an excess of one or more of the principal reagents. Another group of immunoassays are separation-free homogeneous assays in which labeled reagents modulate the label signal upon binding of an assay antibody to the analyte homologs in the sample.
As mentioned above, the assays can be performed either without separation (homogeneous) or with separation (heterogeneous) of any of the assay components or products. Homogeneous immunoassays are exemplified by the EMIT® assay (Siemens Healthcare Diagnostics Inc., Deerfield, IL) disclosed in Rubenstein, et al., U.S. Patent No. 3,817,837, column 3, line 6 to column 6, line 64; the induced luminescence immunoassay ("LOCI® technology") disclosed in U.S. Pat. No. 5,340,716 (Ullman, et al.); immunofluorescence methods such as those disclosed in Ullman, et al., U.S. Patent No. 3,996,345, column 17, line 59, to column 23, line 25; enzyme channeling immunoassays ("ECIA") such as those disclosed in Maggio, et al., U.S. Patent No. 4,233,402, column 6, line 25 to column 9, line 63; the fluorescence polarization immunoassay ("FPIA") as disclosed, for example, in, among others, U.S. Patent No. 5,354,693; enzyme immunoassays such as the enzyme linked immunosorbant assay ("ELISA"). Exemplary of heterogeneous assays are the radioimmunoassay, disclosed in Yalow, et al., J. Clin. Invest. 39:1157 (1960).

Other enzyme immunoassays are the enzyme modulate mediated immunoassay ("EMMIA") discussed by Ngo and Lenhoff, FEBS Lett. (1980) 116:285-288; the substrate labeled fluorescence immunoassay ("SLFIA") disclosed by Oellerich, J. Clin. Chem. Clin. Biochem. (1984) 22:895-904; the combined enzyme donor immunoassays ("CEDIA") disclosed by Khanna, et al., Clin. Chem. Acta (1989) 185:231-240; homogeneous particle labeled immunoassays such as particle enhanced turbidimetric inhibition immunoassays ("PETINIA"), and particle enhanced turbidimetric immunoassay ("PETIA"), etc.; for example.

Other assays include the sol particle immunoassay ("SPIA"), the disperse dye immunoassay ("DIA"); the metalloimmunoassay ("MIA"); the enzyme membrane immunoassays ("EMIA"); luminoimmunoassays ("LIA"); and so forth. Other types of assays include immunosensor assays involving the monitoring of the changes in the optical, acoustic and electrical properties of a reagent upon the binding of an analyte. Such assays include, for example, optical immunosensor assays, acoustic immunosensor assays, semiconductor immunosensor assays, electrochemical transducer immunosensor assays, potentiometric immunosensor assays, and amperometric electrode assays.

Heterogeneous assays usually involve one or more separation steps and can be competitive or non-competitive. A variety of competitive and non-competitive heterogeneous assay formats are disclosed in Davalian, et al., U.S. Pat. No. 5,089,390, column 14, line 25 to column 15, line 9. In an example of a competitive heterogeneous assay, a support having an antibody for analyte bound thereto is contacted with a medium containing the sample suspected of containing the analyte homologs and a an analyte analog that comprises a label. A non-assay antibody in accordance with the principles described herein is incubated with the sample either prior to or concomitantly with the assay antibody on the support. Analyte homologs in the sample compete, for binding to the analyte antibody, with the labeled analyte analog. After separating the support and the medium, the label activity of the support or the medium is determined by conventional techniques and is related to the amount of analyte in the sample. In a variation of the above competitive heterogeneous assay, the support comprises an analyte analog, which competes with analyte homologs of the sample for binding to an assay antibody reagent in accordance with the principles described herein.

In some examples, the sample to be analyzed is subjected to a pretreatment to release analyte from endogenous binding substances such as, for example, plasma or serum proteins that bind the analyte. The release of the analyte from endogenous binding substances may be carried out, for example, by addition of a digestion agent or a releasing agent or a combination of a digestion agent and a releasing agent used sequentially. The digestion agent is one that breaks down the endogenous binding substances so that they can no longer bind the analyte. Such agents include, but are not limited to, proteinase K and proteinase K and protein denaturing agents such as, e.g., detergents (sodium dodecyl sulfate, for example). Releasing agents for releasing the analyte from endogenous binding substances include, by way of illustration and not limitation, acidic denaturing agents such as, for example, salicylic acid, warfarin, sulfonic acids, toluene sulfonic acids, naphthalene sulfonic acid, anilinonaphthalene sulfonic acids (ANS) (including, e.g., 1-anilinonaphthalene-8-sulfonic acid (1,8-ANS) and 8-anilinonapthalene-1-sulfonic acid (8-ANS)), salicylic acids and derivatives of the above.

The conditions such as, for example, duration, temperature, pH and concentration of the releasing agent in the medium for carrying out the digestion and/or releasing actions are dependent on the nature of the analyte, the nature of the endogenous binding substances, the nature of the sample, and the nature of the releasing agent, for example. In general, the conditions are sufficient to achieve the desired effect or function. In some examples in accordance with the principles described herein, an effective concentration of releasing agent is 0.01 to 20 mg/mL, or 0.01 to 10 mg/mL, or 0.01 to 5 mg/mL, or 0.1 to 20 mg/mL, or 0.1 to 10 mg/mL, or 0.1 to 5 mg/mL, or 0.1 to 1 mg/mL. The pretreatment of the sample to release the analyte from endogenous binding substances may be carried out as a separate step prior to conducting an assay or as a first step in an assay. In either case, one or more reagents may be required to stop the action of the digestion agent and/or the releasing agent.

The conditions for conducting an assay on a sample in accordance with the principles described herein include carrying out the assay in an aqueous buffered medium at a moderate pH, generally that which provides optimum assay sensitivity. The aqueous medium may be solely water or may include from 0.1 to 40 volume percent of a cosolvent. The pH for the medium will be in the range of 4 to 11, or in the range of 5 to 10, or in the range of 6.5 to 9.5, for example. The pH will usually be a compromise between optimum binding of the binding members of any specific binding pairs, the pH optimum for other reagents of the assay such as members of the signal producing system, and so forth. Various buffers may be used to achieve the desired pH and maintain the pH during the assay. Illustrative buffers include, by way of illustration and not limitation, borate, phosphate, carbonate, TRIS, barbital, PIPES, HEPES, MES, ACES, MOPS, and BICINE, for example. The particular buffer employed is not critical, but in an individual assay one or another buffer may be preferred.

Various ancillary materials may be employed in the assay methods. For example, in addition to buffers the medium may comprise stabilizers for the medium and for the reagents employed. In some embodiments, in addition to these additives, proteins may be included, such as, for example, albumins; organic solvents such as, for example, formamide; quaternary ammonium salts; polyanions such as, for example, dextran sulfate; binding enhancers, for example, polyalkylene glycols; polysaccharides such as, for example, dextran or trehalose. The medium may also comprise agents for preventing the formation of blood clots. Such agents are well known in the art and include, but are not limited to, EDTA, EGTA, citrate, heparin, for example. The medium may also comprise one or more preservatives such as, but not limited to, sodium azide, neomycin sulfate, PROCLIN® 300, Streptomycin, for example. The medium may additionally comprise one or more surfactants. Any of the above materials, if employed, is present in a concentration or amount sufficient to achieve the desired effect or function.

One or more incubation periods may be applied to the medium at one or more intervals including any intervals between additions of various reagents employed in an assay including those mentioned above. The medium is usually incubated at a temperature and for a time sufficient for binding of various components of the reagents and binding of the analyte in the sample to occur such as, for example, the binding of a non-assay antibody to an analyte homolog or the binding of an assay antibody to analyte homologs. Moderate temperatures are normally employed for carrying out the method and usually constant temperature, preferably, room temperature, during the period of the measurement. In some examples, incubation temperatures range from 5° to 99°C, or from 15°C to 70°C, or from 20°C to 45°C, for example. The time period for the incubation, in some examples, is 0.2 seconds to 24 hours, or 1 second to 6 hours, or 2 seconds to 1 hour, or 1 minute to 15 minutes, for example. The time period depends on the temperature of the medium and the rate of binding of the various reagents, which is determined by the association rate constant, the concentration, the binding constant and dissociation rate constant.

Many assays discussed herein use a signal producing system, which may have one or more components, at least one component being a label. The signal producing system generates a signal that relates to the presence of analyte homologs in a sample. The signal producing system includes all of the reagents required to produce a measurable signal. Other components of the signal producing system may be included in a developer solution and can include, but are not limited to, substrates, enhancers, activators, chemiluminescent compounds, cofactors, inhibitors, scavengers, metal ions, and specific binding substances required for binding of signal generating substances, for example. Other components of the signal producing system may be coenzymes, substances that react with enzymic products, other enzymes and catalysts, for example. The signal producing system provides a signal detectable by external means, by use of electromagnetic radiation, desirably by visual examination. Exemplary signal-producing systems are described in U.S. Patent No. 5,508,178.

The term "label" includes poly(amino acid) labels and non-poly(amino acid) labels. The term "poly(amino acid) label moieties" includes labels that are proteins such as, but not limited to, enzymes, antibodies, peptides, and immunogens, for example. With label proteins such as, for example, enzymes, the molecular weight range will be from 10,000 to 600,000, or from 10,000 to 300,000 molecular weight. There is usually at least one compound in accordance with the principles described herein (analog group) per 200,000 molecular weight, or at least 1 per 150,000 molecular weight, or at least 1 per 100,000 molecular weight, or at least 1 per 50,000 molecular weight, for example, of the protein. In the case of enzymes, the number of analog groups is usually from 1 to 20, 2 to 15, 3 to 12, or 6 to 10.

Enzymes include, by way of illustration and not limitation, redox enzymes such as, for example, dehydrogenases, e.g., glucose-6-phosphate dehydrogenase and lactate dehydrogenase; enzymes that involve the production of hydrogen peroxide and the use of the hydrogen peroxide to oxidize a dye precursor to a dye such as, for example, horseradish peroxidase, lactoperoxidase and microperoxidase; hydrolases such as, for example, alkaline phosphatase and β-galactosidase; luciferases such as, for example firefly luciferase, and bacterial luciferase; transferases; combinations of enzymes such as, but not limited to, saccharide oxidases, e.g., glucose and galactose oxidase, or heterocyclic oxidases, such as uricase and xanthine oxidase, coupled with an enzyme that employs hydrogen peroxide to oxidize a dye precursor, that is, a peroxidase such as horseradish peroxidase, lactoperoxidase or microperoxidase, for example.

The term "non-poly(amino acid) labels" includes those labels that are not proteins. The non-poly(amino acid) label is capable of being detected directly or is detectable through a reaction that produces a detectable signal. The non-poly(amino acid) label can be isotopic or non-isotopic and can be, by way of illustration and not limitation, a radioisotope, a luminescent compound (which includes, but is not limited to fluorescent compounds and chemiluminescent compounds, for example), a polynucleotide coding for a catalyst, a promoter, a dye, a coenzyme, an enzyme substrate, a radioactive group, and an amplifiable polynucleotide sequence, for example.

In some examples one member of the signal producing system is a small organic molecule, which refers to a molecule of molecular weight of 200 to 2,000, or 200 to 1,500, or 200 to 1,000, or 200 to 500. Such small organic molecules include, but are not limited to, biotin, fluorescent molecules (such as fluorescein and rhodamine, for example), chemiluminescent molecules and dinitrophenol, for example. A binding partner for a small organic molecule is a molecule that specifically recognizes and binds to the small molecule. Binding partners for a small molecule are defined by the nature of the small molecule and include, but are not limited to, avidin, streptavidin, antibody for the small organic molecule (which include, but are not limited to, antibody for a fluorescent molecule (such as antibody for fluorescein and antibody for rhodamine, for example), antibody for a chemiluminescent molecule, antibody for dinitrophenol, for example.

In some examples of assays, a support is utilized. The support may be comprised of an organic or inorganic, solid or fluid, water insoluble material and which may be transparent or partially transparent. The support can have any of a number of shapes, such as, but not limited to, a particle (particulate support) including bead, a film, a membrane, a tube, a well, a strip, a rod, a fiber, or a planar surface such as, e.g., a plate or paper, for example. The support may or may not be suspendable in the medium in which it is employed. Examples of suspendable supports are polymeric materials such as latex, lipid bilayers or liposomes, oil droplets, cells and hydrogels, and magnetic particles, for example. Other support compositions include polymers, such as, by way of illustration and not limitation, nitrocellulose, cellulose acetate, poly (vinyl chloride), polyacrylamide, polyacrylate, polyethylene, polypropylene, poly(4 methylbutene), polystyrene, polymethacrylate, poly(ethylene terephthalate), nylon, poly(vinyl butyrate), for example, either used by themselves or in conjunction with other materials. The support may or may not be further labeled with a dye, catalyst or other detectable group, for example.

In some examples, the support may be a particle. The particles have an average diameter of at least 0.02 microns and not more than 100 microns. In some examples, the particles have an average diameter from 0.05 microns to 20 microns, or from 0.3 microns to 10 microns. The particle may be organic or inorganic, swellable or non-swellable, porous or non-porous, preferably of a density approximating water, generally from 0.7 g/mL to 1.5 g/mL, and composed of material that can be transparent, partially transparent, or opaque. The particles can be biological materials such as cells and microorganisms, e.g., erythrocytes, leukocytes, lymphocytes, hybridomas, streptococcus, Staphylococcus aureus, and E. coli, viruses, for example. The particles can also be particles comprised of organic and inorganic polymers, liposomes, latex particles, magnetic or non-magnetic particles, phospholipid vesicles, chylomicrons, lipoproteins, and the like. In some examples, the particles are chromium dioxide (chrome) particles or latex particles.

Chemiluminescent particles are particles that have associated therewith a chemiluminescent compound. The phrase "associated therewith" as used herein means that a compound such as, for example, a chemiluminescent compound and a particle may be associated by direct or indirect bonding, adsorption, absorption, incorporation, or solution, for example. Examples of chemiluminescent compounds that may be utilized are those set forth in U.S. Patent Nos. 5,340,716 and 6,251,581. In some examples in accordance with the principles described herein, the chemiluminescent compound is a photoactivatable substance that undergoes a chemical reaction upon direct or sensitized excitation by light or upon reaction with singlet oxygen to form a metastable reaction product that is capable of decomposition with the simultaneous or subsequent emission of light, usually within the wavelength range of 250 to 1200 nm. The term "photoactivatable" includes "photochemically activatable". In some examples, the chemiluminescent compounds are those that react with singlet oxygen to form dioxetanes or dioxetanones. The latter are usually electron rich olefins. Exemplary of such electron rich olefins are enol ethers, enamines, 9-alkylidene-N-alkylacridans, arylvinylethers, dioxenes, arylimidazoles, 9-alkylidene-xanthanes and lucigenin. Other compounds include luminol and other phthalhydrazides and chemiluminescent compounds that are protected from undergoing a chemiluminescent reaction by virtue of their being protected by a photochemically labile protecting group, such compounds including, for example, firefly luciferin, aquaphorin, and luminol. Examples of such chemiluminescent compounds that may be utilized are those set forth in U.S. Patent No. 5,709,994.

Sensitizer particles are particles that have associated therewith a sensitizer compound, which includes, but is not limited to, a photosensitizer compound. Examples of sensitizer compounds that may be utilized are those set forth in U.S. Patent Nos. 5,340,716 and 6,251,581.

A photosensitizer is a sensitizer for generation of singlet oxygen usually by excitation with light. In some examples, the photosensitizer absorbs at a longer wavelength than the chemiluminescent compound and has a lower energy triplet than the chemiluminescent compound. The photosensitizer can be photoactivatable (e.g., dyes and aromatic compounds). The photosensitizer is usually a compound comprised of covalently bonded atoms, usually with multiple conjugated double or triple bonds. The compound should absorb light in the wavelength range of 200-1100 nm, usually 300-1000 nm, preferably 450-950 nm. Typical photosensitizers include, but are not limited to, acetone, benzophenone, 9-thioxanthone, eosin, 9,10-dibromoanthracene, methylene blue, metallo-porphyrins (e.g., hematoporphyrin), phthalocyanines, chlorophylls, rose bengal, buckminsterfullerene, for example, and derivatives of these compounds. Examples of other photosensitizers are enumerated in N.J. Turro, "Molecular Photochemistry", page 132, W. A. Benjamin Inc., N.Y. 1965. The photosensitizer assists photoactivation where activation is by singlet oxygen. Usually, the photosensitizer absorbs light and the thus formed excited photosensitizer activates oxygen to produce singlet oxygen, which reacts with the chemiluminescent compound to give a metastable luminescent intermediate.

Some known assays utilize a signal producing system (sps) that employs first and second sps members. The sps members may be related in that activation of one member of the sps produces a product such as, for example, light or an activated product, which results in activation of another member of the sps.

In an example of such an assay, the sps members comprise a sensitizer such as, for example, a photosensitizer, and a chemiluminescent composition that includes a chemiluminescent compound where activation of the sensitizer results in a product that activates the chemiluminescent composition. The second sps member usually generates a detectable signal that relates to the amount of bound and/or unbound sps member, i.e., the amount of sps member bound or not bound to the analyte being detected. In some examples in accordance with the principles described herein, one of either the sensitizer reagent or the chemiluminescent reagent comprises an antibody reagent in accordance with the principles described herein.

The concentration of the analyte homologs in a sample that may be assayed generally varies from 10⁻⁵ to 10⁻¹⁷ M, or from 10⁻⁶ to 10⁻¹⁴ M, for example. Considerations such as whether the assay is qualitative, semi-quantitative or quantitative (relative to the amount of the analyte present in the sample), the particular detection technique and the expected concentration of the analyte normally determine the concentrations of the various reagents.

The concentrations of the various reagents in the assay medium will generally be determined by the concentration range of interest of the analyte homologs and the nature of the assay, for example. However, the final concentration of each of the reagents is normally determined empirically to optimize the sensitivity of the assay over the range of interest. That is, a variation in concentration of analyte that is of significance should provide an accurately measurable signal difference. Considerations such as the nature of the signal producing system and the nature of the analyte homologs normally determine the concentrations of the various reagents.

As mentioned above, the sample and reagents are provided in combination in the medium. While the order of addition to the medium may be varied, there will be certain preferences for some embodiments of the assay formats described herein. The simplest order of addition, of course, is to add all the materials simultaneously and determine the effect that the assay medium has on the signal as in a homogeneous assay. Alternatively, each of the reagents, or groups of reagents, can be combined sequentially. In some embodiments, an incubation step may be involved subsequent to each addition as discussed above. In heterogeneous assays, washing steps may also be employed after one or more incubation steps.

### Specific Examples of Methods in Accordance with the Principles Described Herein

As discussed above, methods in accordance with the principles described herein are directed to determining in a sample a total amount of two or more analyte homologs in a sample suspected of containing the analyte homologs. A total amount of the analyte homologs is measured by conducting an assay on a sample using an assay receptor such as, for example, an assay antibody. Prior to or simultaneously therewith, the sample is incubated with a non-assay antibody as discussed in more detail above. In the examples set forth below, both the assay antibody and the non-assay antibody are monoclonal antibodies prepared and screened by one or more of the procedures described above.

In the following particular examples, the analyte homologs are vitamin D₂ and vitamin D₃ by way of illustration and not limitation. Some examples in accordance with the principles described herein are directed to methods of determining a total amount of vitamin D₂ and vitamin D₃ in a sample suspected of containing vitamin D homologs and may be referred to herein as "assays for vitamin D." As used herein in reference to the specific assays discussed below, the term "vitamin D homolog(s)" refers to vitamin D₂ and vitamin D₃.

In one example, an induced luminescence immunoassay may be employed. The induced luminescence immunoassay is referred to in U.S. Pat. No. 5,340,716 (Ullman). In one approach, the assay uses a particle having associated therewith a photosensitizer where a vitamin D analog is bound to the particle (particle-analog reagent). The chemiluminescent reagent comprises an assay antibody that exhibits sufficient assay binding affinity for each of the vitamin D₂ and vitamin D₃ homologs. In this example, the assay antibody exhibits substantially equivalent binding affinity for the vitamin D₂ and vitamin D₃ homologs. However, the sample is pretreated with a releasing agent and the vitamin D₂ homolog is released from endogenous binding substances in an amount greater than that for the vitamin D₃ homolog. In accordance with the principles described herein, a non-assay antibody prepared as described above is incubated with the medium comprising the released homologs. The non-assay antibody exhibits a preferential binding affinity for the vitamin D₂ homolog, which is released in excess by the releasing agent. The amount of the non-assay antibody is sufficient to adjust the amount of signal obtained in the assay to compensate for the amount of the vitamin D₂ homolog released in excess of the amount of vitamin D₃ homolog. Treatment of the medium comprising the vitamin D₂ and vitamin D₃ homologs may be carried out in a separate step or may be carried out in conjunction with the assay using an assay antibody.

In the above example, the assay antibody is linked to a small molecule, which is bound to a binding partner for the small molecule on a chemiluminescent particle. This chemiluminescent reagent may be pre-formed or formed *in situ.* The vitamin D analyte homologs compete with the particle-analog reagent for binding to the assay antibody for vitamin D. If the vitamin D analyte homologs are present, the fewer is the number of molecules of particle-analog reagent that come into close proximity with the chemiluminescent reagent. Therefore, there will be a decrease in the assay signal. The photosensitizer generates singlet oxygen and activates the chemiluminescent reagent when the two labels are in close proximity. The activated chemiluminescent reagent subsequently produces light, where a decrease in signal is observed in the presence of the analyte. The amount of light produced is related to the amount of the complex formed, which in turn is related to the total amount of the vitamin D₂ and vitamin D₃ homologs present in the sample.

In another particular example of an induced luminescence immunoassay using vitamin D as an example, by way of illustration and not limitation, the assay uses a particle having associated therewith a chemiluminescent compound where a vitamin D analog is bound to the particle (particle-analog reagent). A photosensitizer reagent is employed that comprises an assay antibody that exhibits a binding affinity for the vitamin D₂ homolog that is greater than its binding affinity for the vitamin D₃ homolog. The assay antibody is linked to a small molecule that is in turn bound to a binding partner for the small molecule on a chemiluminescent particle.

In this example, the assay antibody exhibits a binding affinity for the vitamin D₂ homolog that is greater than its binding affinity for the vitamin D₃ homologs. The sample is pretreated with a releasing agent, which provides for release of substantially equal amounts of the vitamin D homologs from endogenous binding substances. In accordance with the principles described herein, a non-assay antibody prepared as described above is incubated with the medium comprising the released homologs. The non-assay antibody exhibits a preferential binding affinity for the vitamin D₂ homolog, for which the assay antibody has a greater binding affinity. The amount of the non-assay antibody is sufficient to adjust the amount of signal obtained in the assay to compensate for the amount of the vitamin D₂ homolog that is bound by the assay antibody in an amount greater than that for the amount of vitamin D₃ homolog. Treatment of the medium comprising the vitamin D₂ and vitamin D₃ homologs is carried out in conjunction with the assay using an assay antibody.

Both the vitamin D₂ and vitamin D₃ homologs compete with the particle-analog reagent for binding to the assay antibody for vitamin D. If the vitamin D analyte homologs are present, the fewer is the number of molecules of particle-analog reagent that come into close proximity with the photosensitizer reagent. Therefore, there will be a decrease in the assay signal. The photosensitizer generates singlet oxygen and activates the chemiluminescent compound of the particle-analog reagent when the two labels are in close proximity. The activated chemiluminescent compound subsequently produces light, where a decrease in signal is observed in the presence of the analyte. The amount of light produced is related to the amount of the complexes formed, which in turn is related to the amount of both the vitamin D₂ and vitamin D₃ homologs present in the sample.

In another particular example of an induced luminescence assay using vitamin D by way of illustration and not limitation, a photosensitizer particle is employed that is conjugated to a binding partner for a small molecule such as, for example, avidin or streptavidin (which are binding partners for biotin). An assay antibody reagent comprises biotin linked to an assay antibody that binds to both of the vitamin D analyte homologs is employed. A chemiluminescent reagent is employed as part of the detection system.

In this example, the assay antibody exhibits a binding affinity for the vitamin D₂ homolog that is greater than its binding affinity for the vitamin D₃ homologs. Furthermore, the sample is pretreated with a releasing agent and the vitamin D₂ homolog is released from endogenous binding substances in an amount greater than that for the vitamin D₃ homolog. In accordance with the principles described herein, a non-assay antibody prepared as described above is incubated with the medium comprising the released homologs. The non-assay antibody exhibits a preferential binding affinity for the vitamin D₂ homolog, for which the assay antibody has a greater binding affinity and which is released from endogenous binding substances in an amount greater than that for the vitamin D₃ homolog. The amount of the non-assay antibody is sufficient to adjust the amount of signal obtained in the assay to compensate for the amount of the vitamin D₂ homolog that is bound by the assay antibody in an amount greater than that for the amount of vitamin D₃ homolog and for the amount of the vitamin D₂ homolog that is released in excess of that amount of vitamin D₃ homolog released. Treatment of the medium comprising the vitamin D₂ and vitamin D₃ homologs is carried out in conjunction with the assay using an assay antibody.

The reaction medium comprising the above reagents is incubated to allow the avidin or streptavidin of the photosensitizer particles to bind to the biotin of the assay antibody reagent by virtue of the binding between avidin and biotin and to also allow the specific binding between the assay antibody of the assay antibody reagent, which is now attached to the photosensitizer particles, to bind to the analyte homologs of the sample and to the analyte that is part of the chemiluminescent reagent. The incubation also allows the binding between the non-assay antibody and the vitamin D₂ analyte homolog. Then, the medium is irradiated with light to excite the photosensitizer, which is capable in its excited state of activating oxygen to a singlet state. Because less of the chemiluminescent reagent is now in close proximity to the photosensitizer because of the presence of the analyte homologs, there is less activation of the chemiluminescent reagent by the singlet oxygen and less luminescence. The medium is then examined for the presence and/or the amount of luminescence or light emitted, the presence thereof being related to the presence and/or amount of the analyte homologs where a decrease in signal is observed in the presence of the analyte. The amount of light produced is related to the amount of the complex formed, which in turn is related to the amount of both the vitamin D₂ and vitamin D₃ homologs present in the sample.

Another example of an assay format for detection of vitamin D, by way of illustration and not limitation, in a sample is the ACMIA assay format. For the ACMIA assay format, chrome particles, which are coated with vitamin D or a vitamin D analog (chrome particle reagent), are employed as a first component. A second component is an assay antibody reagent that comprises a first assay antibody for the vitamin D₂ and vitamin D₃ homologs. In the antibody reagent, the first assay antibody is linked by means of a linking group to a reporter enzyme (for example, β-galactosidase) to form an assay antibody-enzyme conjugate.

In this example, the assay antibody exhibits substantially equivalent binding affinity for the vitamin D₂ and vitamin D₃ homologs. However, the sample is pretreated with a releasing agent and the vitamin D₂ homolog is released from endogenous binding substances in an amount greater than that for the vitamin D₃ homolog. In accordance with the principles described herein, a non-assay antibody prepared as described above is incubated with the medium comprising the released homologs. The non-assay antibody exhibits a preferential binding affinity for the vitamin D₂ homolog, which is released in excess by the releasing agent. The amount of the non-assay antibody is sufficient to adjust the amount of signal obtained in the assay to compensate for the amount of the vitamin D₂ homolog released in excess of the amount of vitamin D₃ homolog. Treatment of the medium comprising the vitamin D₂ and vitamin D₃ homologs may be carried out in a separate step or may be carried out in conjunction with the assay using an assay antibody.

A medium comprising the sample is treated with a second assay antibody reagent, which comprises a second assay antibody that exhibits substantially equivalent assay binding affinity for each of the vitamin D analyte homologs; the second assay antibody binds to vitamin D homologs in the sample. The medium also contains a non-assay antibody as discussed above. The antibody-enzyme conjugate is mixed with sample in the medium to allow the vitamin D analyte homologs to bind to the first assay antibody. Next, the chrome particle reagent is added to bind up any excess antibody-enzyme conjugate. Then, a magnet is applied, which pulls all of the chrome particles and excess antibody-enzyme out of the suspension, and the supernatant is transferred to a final reaction container. The substrate of the reporter enzyme is added to the final reaction container, and the enzyme activity is measured spectrophotometrically as a change in absorbance over time. The amount of this signal is related to the amount of both homologic forms of the vitamin D in the sample.

Another example of an assay for homologic forms of vitamin D (by way of illustration and not limitation) in a sample is an acridinium ester label immunoassay using paramagnetic particles as a solid phase (ADVIA immunoassay). The detection system employed for this example of a vitamin D assay includes a small molecule-labeled vitamin D (capture moiety) as the small molecule conjugate or capture conjugate, binding partner for the small molecule-coated paramagnetic latex particles as a solid phase (SP), and an acridinium ester labeled assay antibody for the vitamin D₂ and vitamin D₃ homologs (detection antibody). The small molecule may be, for example, biotin or fluorescein and the respective binding partner may be streptavidin or antibody for fluorescein. The vitamin D analog may be linked to the small molecule directly or through a linking group such as, for example, a protein, e.g., bovine serum albumin (BSA). The vitamin D₂ and vitamin D₃ homologs in a patient sample compete with the vitamin D analog of the capture moiety for binding to the acridinium ester labeled detection anti-vitamin D assay antibody.

The sample suspected of containing vitamin D homologs is subjected to a pretreatment with 1,8-ANS. In this example, the assay antibody exhibits substantially equivalent binding affinity for the vitamin D₂ and vitamin D₃ homologs. However, the as a result of the pretreatment with 1,8-ANS, the vitamin D₂ homolog is released from endogenous binding substances in an amount greater than that for the vitamin D₃ homolog. In accordance with the principles described herein, a non-assay antibody prepared as described above is incubated with the medium comprising the released homologs. The non-assay antibody exhibits a preferential binding affinity for the vitamin D₂ homolog, which is released in excess by the releasing agent. The amount of the non-assay antibody is sufficient to adjust the amount of signal obtained in the assay to compensate for the amount of the vitamin D₂ homolog released in excess of the amount of vitamin D₃ homolog. Treatment of the medium comprising the vitamin D₂ and vitamin D₃ homologs with the non-assay antibody may be carried out in a separate step or may be carried out in conjunction with the assay using an assay antibody. The assay may be carried out using an ADVIA CENTAUR®, ADVIA CENTAUR® XP or ADVIA CENTAUR® CP apparatus (Siemens Healthcare Diagnostics Inc., Newark DE) in accordance with the manufacturer's directions supplied therewith.

Another example of an assay for an analyte in accordance with the principles described herein is an acridinium ester label immunoassay using paramagnetic particles as a solid phase (ADVIA immunoassay). The detection system employed for this example of an assay for vitamin D homologs includes an assay antibody reagent comprising an assay antibody and a small molecule linked to the assay antibody (capture antibody) as the a capture conjugate, paramagnetic latex particles as a solid phase (SP) coated with a binding partner for the small molecule of the assay antibody reagent, and an acridinium ester labeled vitamin D analyte analog (detection hapten). The acridinium ester label may be directly bound to the analyte analog to form the detection hapten or a linking group may be employed including, for example, a protein such as, e.g., BSA. The vitamin D₂ and vitamin D₃ homologs of a sample compete with the acridinium ester labeled detection hapten for binding with the assay antibody.

In this example, the assay antibody exhibits a binding affinity for the vitamin D₂ homolog that is greater than its binding affinity for the vitamin D₃ homologs. The sample suspected of containing the vitamin D₂ and vitamin D₃ homologs is subjected to a pretreatment with one or more of a releasing agent and a digestion agent, which provides for release of substantially equal amounts of the vitamin D homologs from endogenous binding substances. In accordance with the principles described herein, a non-assay antibody prepared as described above is incubated with the medium comprising the released homologs. The non-assay antibody exhibits a preferential binding affinity for the vitamin D₂ homolog, for which the assay antibody has a greater binding affinity. The amount of the non-assay antibody is sufficient to adjust the amount of signal obtained in the assay to compensate for the amount of the vitamin D₂ homolog that is bound by the assay antibody in an amount greater than that for the amount of vitamin D₃ homolog. Treatment of the medium comprising the vitamin D₂ and vitamin D₃ homologs is carried out in conjunction with the assay using the assay antibody reagent.

The above assay may be carried out using an ADVIA CENTAUR®, ADVIA CENTAUR® XP or ADVIA CENTAUR® CP apparatus (Siemens Healthcare Diagnostics Inc., Newark DE) in accordance with the manufacturer's directions supplied therewith. In variations of the above acridinium ester assays, the small molecule may be, for example, biotin or fluorescein and the binding partners for the small molecule may be, for example, avidin or streptavidin or antibody for fluorescein, respectively.

### Examination Step

In one step of an assay method, the medium is examined for the presence of a complex comprising homologic forms of an analyte and antibody for an analyte in accordance with the principles described herein. The amount of the complexes indicates the amount of homologic forms of the analyte in the sample.

The phrase "measuring the amount of analyte" refers to the quantitative, semiquantitative and qualitative determination of the homologic forms of an analyte. Methods that are quantitative, semiquantitative and qualitative, as well as all other methods for determining the analyte, are considered to be methods of measuring the amount of the analyte. For example, a method, which merely detects the presence or absence of the analyte in a sample suspected of containing the analyte, is considered to be included within the scope of the present invention. The terms "detecting" and "determining," as well as other common synonyms for measuring, are contemplated within the scope of the present invention.

In many embodiments the examination of the medium involves detection of a signal from the medium. The presence and/or amount of the signal is related to the presence and/or amount of the homologic forms of an analyte in the sample. The particular mode of detection depends on the nature of the signal producing system. As discussed above, there are numerous methods by which a label of a signal producing signal can produce a signal detectable by external means. Activation of a signal producing system depends on the nature of the signal producing system members.

Temperatures during measurements generally range from 10°C to 70°C or from 20°C to 45°C, or 20°C to 25°C, for example. In one approach standard curves are formed using known concentrations of vitamin D analyte as described above. Calibrators and other controls may also be used.

Luminescence or light produced from any label can be measured visually, photographically, actinometrically, spectrophotometrically, such as by using a photomultiplier or a photodiode, or by any other convenient means to determine the amount thereof, which is related to the amount of analyte homologs in the medium. The examination for presence and/or amount of the signal also includes the detection of the signal, which is generally merely a step in which the signal is read. The signal is normally read using an instrument, the nature of which depends on the nature of the signal. The instrument may be, but is not limited to, a spectrophotometer, fluorometer, absorption spectrometer, luminometer, and chemiluminometer, for example.

### Kits comprising reagents for conducting assays

Kits for conducting assays on portions of a sample suspected of containing homologic forms of an analyte may be prepared. The kits comprise antibody reagents that include at least one assay antibody for the analyte homologs in a sample. The kits further comprise a non-assay receptor in accordance with the principles described herein. An amount of the non-assay receptor is sufficient to achieve an adjustment of the contribution of one of two analyte homologs to the signal. The kit may further include other reagents for performing the assay, the nature of which depend upon the particular assay format.

The reagents may each be in separate containers or various reagents can be combined in one or more containers depending on the cross-reactivity and stability of the reagents. The kit can further include other separately packaged reagents for conducting an assay such as additional specific binding pair members, signal producing system members, and ancillary reagents, for example.

The relative amounts of the various reagents in the kits can be varied widely to provide for concentrations of the reagents that substantially optimize the reactions that need to occur during the present methods and further to optimize substantially the sensitivity of an assay. Under appropriate circumstances one or more of the reagents in the kit can be provided as a dry powder, usually lyophilized, including excipients, which on dissolution will provide for a reagent solution having the appropriate concentrations for performing a method or assay using a compound reagent in accordance with the principles described herein. The kit can further include a written description of a method utilizing reagents that include a compound reagent in accordance with the principles described herein.

The designation "first" and "second" as used herein is completely arbitrary and is not meant to suggest any order or ranking among moieties referred to or any order of addition of moieties in the present methods.

The phrase "at least" as used herein means that the number of specified items may be equal to or greater than the number recited.

The following discussion is directed to specific examples in accordance with the principles described herein by way of illustration and not limitation; the specific examples are not intended to limit the scope of the present disclosure and the appended claims. Numerous modifications and alternative compositions, methods, and systems may be devised without departing from the spirit and scope of the present disclosure.

### EXAMPLES

Unless otherwise indicated, materials in the experiments below may be purchased from the Sigma-Aldrich Chemical Corporation (St. Louis MO) or Fluka Chemical Corporation (Milwaukee WI). Parts and percentages disclosed herein are by weight to volume unless otherwise indicated.

### Definitions:

- mg =: milligram
- g =: gram(s)
- ng =: nanogram(s)
- mL =: milliliter(s)
- µL =: microliter(s)
- µmol =: micromolar
- °C =: degrees Centigrade
- min =: minute(s)
- sec =: second(s)
- hr =: hour(s)
- w/v =: weight to volume
- v/v =: volume to volume
- EDA =: ethylenediamine
- EDAC =: N-(3-Dimethylaminopropyl)-N'-ethylcarbodiimide hydrochloride
- sulfoNHS or SNHS =: sulfo N-hydroxysuccinimide
- TLC = thin: layer chromatography
- HPLC =: high performance liquid chromatography
- EDTA =: ethylenediaminetetraacetate
- PEG =: polyethylene glycol
- EtOAc =: ethyl acetate
- DMF =: dimethylformamide
- DMSO =: dimethylsulfoxide
- MeOP =: 1-methoxy-2-propanol
- MES =: 2-(N-morpholino)ethanesulfonic acid
- DI =: distilled
- UPA =: Ultra Particle Analyzer
- LOCI =: luminescent oxygen channeling immunoassay
- Ab =: antibody

### Preparation of biotinylated assay antibody that exhibits substantially equimolar assay binding affinity for both 25-hydroxy vitamin D₂ and 25-hydroxy vitamin D₃

A solution (0.8 mL at 2.63 mg/mL) of vitamin D antibody 5H10 (sheep monoclonal from Bioventix, Farnham, Surrey, UK) in 10 mM PO₄, 300 mM NaCl, pH 7.0 was mixed with 43.2 µL of an aqueous solution (2.0 mg/mL) of NHS-dPEG®4-biotin (Quanta Biodesign Ltd., Powell OH, part number 10200). The amount of biotinylation reagent added represents a 10-fold molar challenge of the biotinylating agent with the antibody. The reaction mixture was incubated at room temperature for 3 hr and then the reaction was quenched by addition of 80 µL of 0.5 M TRIS. The reaction mixture was subjected to buffer exchange with 10 mM PO₄, 300 mM NaCl, pH 7.0 in an AMICON® (YM10) device until absorption at 260 nm of the effluent was ≤0.03. The antibody solution (1.04 mL at 2.1 mg/mL protein) was mixed with 10 µL of PROCLIN® 300 and 10 µL of an aqueous solution of neomycin sulfate (10 mg/mL) filtered using a 0.2 um ACRODISC® syringe filter (Pall Corporation) and was stored at 2-8°C.

### Preparation of EPRM-EDA beads

EPRM beads (2000 mg, 20.0 mL) are added to a 40-mL vial. The EPRM beads are prepared by a procedure similar to that described in U.S. Patent No. 7,179,660 and the chemiluminescent compound is 2-(4-(N,N, di- tetradecyl)-anilino-3-phenyl thioxene with europium chelate. EDA (800 mg, 890 µL) is combined with 10 mL MES pH 6 buffer (the "Buffer") and 4.2 mL 6N HCl. The pH of the mixture is, or is adjusted to be, 6.9. The EDA solution is added to the EPRM beads with vortexing and the mixture is rocked at room temperature for 15 minutes. Sodium cyanoborohydride (400 mg) is combined in a 15 mL vial with 10 mL DI water and the combination is added to the bead mixture from above. The mixture is shaken at 37 °C for 18-20 hours. The beads are transferred to six 40 mL centrifuge tubes. MES buffer is added to bring the volume to 35 mL and the mixture is centrifuged at 19,000 rpm for 30 min. The supernatant is decanted and the beads are re-suspended in 2 mL of the Buffer with a stir-rod and additional Buffer is added to 35 mL. The mixture is sonicated at 18 Watts power for 30 sec, using ice to keep the mixture cold. The wash/sonication step is performed 4 times to remove all activation chemical. After the last MES Buffer centrifugation, 2 mL of the Buffer containing 5% MeOP and 0.1% Tween® 20 (the "second Buffer") is added to the tubes for the re-suspension step. Additional second buffer is added to 35 mL before sonication. The bead suspension is centrifuged at 19,000 rpm for 30 min. The supernatant is discarded. The final sonication used 12 mL of the second Buffer in each tube to give a 25 mg/mL dilution. Particle size is 277 nm as determined on a UPA instrument.

The EPRM chemibead is prepared in a manner similar to the method described in U.S. Patent No. 6,153,442 and U.S. Patent Application Publication No. 20050118727A. The EPRM chemibead comprises an aminodextran inner layer and a dextran aldehyde outer layer having free aldehyde functionalities. See, for example, U.S. Patent Nos. 5,929,049, 7,179,660 and 7,172,906. The reaction is carried out at a temperature of 0 to 40°C for a period of 16 to 64 hours at a pH of 5.5 to 7.0, or 6, in a buffered aqueous medium employing a suitable buffer such as, for example, MES. The reaction is quenched by addition of a suitable quenching agent such as, for example, carboxymethoxyamine hemihydrochloride (CMO), and subsequent washing of the particles.

Aldehyde groups on the outer dextran aldehyde layer are reacted with ethylene diamine under reductive amination conditions to form reagent EPRM-EDA having pendant moieties comprising an ethylene chain and a terminal amine group. The reductive amination conditions include the use of a reducing agent such as, for example, a metal hydride. The reaction is carried out in an aqueous medium at a temperature during the reaction of 20°C to 100°C for a period of 1 hour to 48 hours.

### Synthesis of 25-OH Vitamin D₃ 3-carbamate (25-OH Vitamin D₂ 3-carbamate)

A mixture of 22 mg (55 µmol) 25-OH VD₃ purchased from ChemReagents.com, Sugarland TX, 100 mg (420 µmol) disuccinimidyl carbonate (DSC), 100µL triethylamine in 1 mL anhydrous acetonitrile in a 5-ml flask (covered with foil) was stirred at room temperature for 18 hr under nitrogen to prepare activated 25-OH VD₃. TLC (EtOAc:Hexane = 2:1) showed no starting material left. A suspension was prepared by adding 150 mg of carboxymethoxylamine hemihydrochloride (CMO), 0.3 ml triethylamine and 1 ml DMF to a 10 ml flask. A solution containing activated 25-OH VD₃ was added dropwise to the CMO suspension with stirring, which was continued for another 18 hr. Vacuum was applied to remove the solvents as much as possible (the heating bath temperature should not be over 50°C). EtOAc (25 ml) was added to the residue, which was washed three times with 2 ml brine. The organic phase was dried with anhydrous Na₂SO₄ and was filtered; solvent was removed using rotavap. Crude product (42 mg) was obtained after drying and was purified by HPLC. Pure product (24 mg) was obtained after being dried under high vacuum. The product was dissolved into 1.2 ml anhydrous DMSO. Aliquots were transferred into vials, which were kept at -70°C.

### Coupling of EPRM-EDA and 25-OH Vitamin D₃ 3-Carbamate to give chemibead reagent

25-OH Vitamin D₃ 3-Carbamate (10 µL of aliquot in DMSO prepared as described above) (0.2 mg) was added to a 2-mL vial. EDAC (6.8 mg) and SNHS (9.4 mg) plus 2.27 mL dry DMSO (3 mg/mL) were added to a 5-mL vial. The EDAC/SNHS solution (190 µL) was combined with the contents of the 2-mL vial from above (1 mg/mL) to prepare activated 25-OH vitamin D₃ 3-carbamate. The mixture was allowed to rotate at room temperature for 18 hr. A 0.4 mL aliquot of a 16% GAFAC® surfactant solution (GAF Corporation, Wayne NJ) (0.15%) was diluted to 1.6% with 3.6 mL DI water.

Vitamin D₃ (8.5 mg) and 850 µL DMSO (10 mg/mL) were combined. To a 10-mL round bottom flask (labeled 3323-064B) equipped with a stir-bar was added 2.0 mL (200MG) EPRM-EDA followed by 400 µL (4 mg) of the Vitamin D₃ solution from above. The mixture stirred overnight at room temperature.

To a 10-mL round bottom flask equipped with a stir-bar was added 2.0 mL (200 mg) EPRM-EDA (prepared as described above) followed by 260 µL 1.6% GAFAC® surfactant solution (0.15%) with moderate stirring. To a small test tube was added 504 µL anhydrous DMSO followed by 60 µL (0.06 mg) activated Vitamin D₃-3-carbamate prepared as described above; and the mixture was added to the EPRM-EDA bead mixture. The total DMSO content of the bead suspension was 20%. The reaction vessel was allowed to stir overnight at room temperature. Then, the beads were washed by means of diafiltration.

Each bead lot was taken up to 20 mL working volume with 10% MeOP/1% GAFAC®/MES pH6 buffer. The mixture was diafiltered with 5 volumes of the buffer and then sonicated with a probe sonicator at 18-21 Watts using ice to keep the mixture cold. The diafiltration/sonication continued through 50 volumes with effluent samples being taken at 35, 40, 45 and 50 volumes. The buffer was changed to LOCI Hapten Wash Buffer (50 mM HEPES, 300 mM NaCl, 1 mM EDTA, 0.01% neomycin sulfate, 0.1% TRITON® 405X and 0.15% PROCLIN® 300, pH 7.2) with 10 volumes being used. The mixture was reduced to 7 mL and a UPA performed. Particle sizes were 3323-064A = 289 nm and 3323-064B = 298 nm. Percent solids were determined and the bead lot was brought up to 10 mg/mL with LOCI Hapten Wash Buffer pH7.2. Yield was 160.4 mg.

### Assay for total amount of 25-hydroxy vitamin D₂ and 25-hydroxy vitamin D₃

Assays were carried out on a DIMENSION® VISTA® analyzer (Siemens Healthcare Diagnostics Inc., Deerfield, IL) following the protocol for a LOCI assay and using calibrator solutions containing varying amounts of 25-hydroxyvitamin D₃. In this example, the assay uses, as a chemiluminescent reagent, the chemibead reagent prepared as described above. The sample suspected of containing vitamin D is subjected to a pretreatment with 5% sodium trichloroacetate and 6% sodium salicylate in HEPES buffer at pH 7.5 (5 mM HEPES, 4.9 mM sodium HEPES, 300 mM sodium chloride, 10% ethylene glycol, 12.26 mM sodium cholate and 0.20% PLURONIC® 25R2).

The conditions for the pretreatment are as follows: 8 µL of serum sample containing 45ng/mL of 25-hydroxy vitamin D₂ or 25-hydroxy vitamin D₃ is incubated with the above pretreatment reagent and biotinylated assay antibody reagent for 18 minutes. Then 25OH D3 coated chemibeads reagent is added to scavenge the unreacted (unbound) biotinylated assay antibody. Next, the streptavidin coated sensibead reagent is then added to generate LOCI signal for the unbound assay antibody. The 25-hydroxy vitamin D concentration is inversely proportional to the LOCI signal. As a result of the pretreatment, an amount of 25-hydroxy vitamin D₂ released from endogenous binding substances and bound to the assay antibody is 63 ng/mL and an amount of 25-hydroxy vitamin D₃ released from endogenous binding substances was 42 ng/mL. As is evident, the amount of 25-hydroxy vitamin D₂ released was greater than the amount of 25-hydroxy vitamin D₃ released.

Sample is reacted with the biotinylated assay antibody reagent prepared as described above and then with the chemibead reagent. Vitamin D antibody 3A10 (mouse monoclonal antibody from Bioventix Inc., Farnham, Surrey, UK) is employed as a non-assay antibody as a solution and the non-assay antibody is present in an amount of 40 µg/mL (or 53 times that of the 5H10 antibody) to compensate for the excess amount of 25-hydroxy vitamin D₂ released over the amount of 25-hydroxy vitamin D₃ released. The chemibeads bind to the fraction of the monoclonal antibody binding sites that is not occupied by analyte from the sample. Subsequently, streptavidin coupled sensitizer beads are added to the reaction mixture. This leads to the formation of chemibead/sensibead pairs whose concentration is inversely related to a concentration of either both forms of the vitamin D (first sample portion) or the epi form of vitamin D (second sample portion). Upon illumination at 680 nm, the sensitizer beads generate singlet oxygen which diffuses into the chemibeads which are paired with sensibeads, reacts with the olefinic dye and triggers a chemiluminescent signal at approximately 612 nm which is inversely related to the analyte concentration.

The streptavidin-sensitizer bead ("sensibead(s)") is prepared using a method analogous to that described in U.S. Patent Nos. 6,153,442, 7.022,529, 7,229,842 and U.S. Patent Application Publication No. 20050118727A. The photosensitizer was bis-(trihexyl)-silicon-t-butyl-phthalocyanine. The concentration of sensibead reagent was 200 µg/mL in HEPES buffer, pH 8.0 containing 150 mM NaCl. The EPRM-EDA-25-OH Vitamin D₃ particle reagent prepared as described above was employed as the "chemibead reagent" at a concentration of 200 µg/mL in HEPES buffer, pH 7.2, containing 150 mM NaCl and 0.1% detergent.

At time t = zero sec, 20 µL biotinylated antibody reagent and 20 µL water were added to a reaction vessel. Sample, 12 µL, was added 21.6 seconds later, followed by 8 µL water. At t = 414.0 seconds, 40 µL chemibead reagent was added followed by 20 mL of water. Sensibead reagent was then dispensed at 457.2 seconds. Measurements were taken 601.2 seconds after initiation of the reaction sequence.

Using the above assay format, assays were carried out on serum samples that contained varying amounts of 25-hydroxy vitamin D₂ and 25-hydroxy vitamin D₃. The above assay was repeated using monoclonal antibodies 5F6 and 6F11 (both sheep monoclonal from Bioventix, Farnham, Surrey, UK), respectively, in place of monoclonal antibody 10H9.

It should be understood that the above-described examples are merely illustrative of some of the many specific examples that represent the principles described herein. Clearly, those skilled in the art can readily devise numerous other arrangements without departing from the scope as defined by the following claims.

## Claims

1. A method of determining in a sample a total amount of 25-hydroxy vitamin D₂ and 25-hydroxy vitamin D₃, the method comprising:
(a) providing in combination in an assay medium:
(i) a sample suspected of containing 25-hydroxy vitamin D₂ and 25-hydroxy vitamin D₃,
(ii) reagents for conducting an assay for 25-hydroxy vitamin D₂ and 25-hydroxy vitamin D₃ wherein the reagents comprise an assay antibody capable of binding to 25-hydroxy vitamin D₂ and 25-hydroxy vitamin D₃ to form complexes and a member of a signal producing system that produces a signal in relation to the amount of 25-hydroxy vitamin D₂ and 25-hydroxy vitamin D₃ complexes, and
(iii) a non-assay antibody that has a greater binding affinity for 25-hydroxy vitamin D₂, wherein an amount of the non-assay antibody is sufficient to achieve an adjustment of the contribution of 25-hydroxy vitamin D₂ to the amount of signal, wherein the amount of the non-assay antibody is 10 µg/mL to 75 µg/mL;
(b) incubating the assay medium under conditions for forming the complexes,
(c) determining the amount of signal from the complexes, and relating the amount of signal to the total amount of 25-hydroxy vitamin D₂ and 25-hydroxy vitamin D₃ in the sample.

2. The method according to claim 1 wherein the member of the signal producing system is a vitamin D analyte analog that comprises a label wherein the vitamin D analog is capable of binding to the assay antibody.

3. The method according to claim 1 wherein reagents for conducting the assay further comprise a particle.

4. The method according to claim 1 wherein the member of the signal producing system comprises a photosensitizer reagent and the assay reagents further comprise a chemiluminescent particle, wherein the photosensitizer reagent preferably comprises a particle.

## Patentansprüche

1. Verfahren zur Bestimmung einer Gesamtmenge von 25-Hydroxyvitamin D₂ und 25-Hydroxyvitamin D₃ in einer Probe, wobei das Verfahren Folgendes umfasst:
(a) Bereitstellen in Kombination in einem Testmedium:
(i) eine Probe, die vermutlich 25-Hydroxyvitamin D₂ und 25-Hydroxyvitamin D₃ enthält,
(ii) Reagentien zur Ausführung eines Tests auf 25-Hydroxyvitamin D₂ und 25-Hydroxyvitamin D₃, wobei die Reagentien einen Testantikörper mit der Fähigkeit zur Bindung an 25-Hydroxyvitamin D₂ und 25-Hydroxyvitamin D₃ unter Bildung von Komplexen und ein Mitglied eines signalproduzierenden Systems, das ein Signal in Beziehung zur Menge von 25-Hydroxyvitamin-D₂- und 25-Hydroxyvitamin-D₃-Komplexen produziert, umfassen, und
(iii) einen Nicht-Testantikörper, der eine größere Bindungsaffinität für 25-Hydroxyvitamin D₂ aufweist, wobei eine Menge des Nicht-Testantikörpers zur Erzielung einer Korrektur des Beitrags von 25-Hydroxyvitamin D₂ zur Menge an Signal hinreichend ist, wobei die Menge des Nicht-Testantikörpers 10 µg/ml bis 75 µg/ml beträgt;
(b) Inkubieren des Testmediums unter Bedingungen für die Bildung der Komplexe,
(c) Bestimmen der Menge an Signal von den Komplexen und Inbeziehungsetzen der Menge an Signal zur Gesamtmenge von 25-Hydroxyvitamin D₂ und 25-Hydroxyvitamin D₃ in der Probe.

2. Verfahren nach Anspruch 1, wobei es sich bei dem Mitglied des signalproduzierenden Systems um ein Vitamin-D-Analytanalog handelt, das eine Markierung umfasst, wobei das Vitamin-D-Analog zur Bindung an den Testantikörper fähig ist.

3. Verfahren nach Anspruch 1, wobei Reagentien zur Ausführung des Tests ferner ein Partikel umfassen.

4. Verfahren nach Anspruch 1, wobei das Mitglied des signalproduzierenden Systems ein Photosensibilisator-Reagens umfasst und die Testreagentien ferner ein Chemilumineszenzpartikel umfassen, wobei das Photosensibilisator-Reagens vorzugsweise ein Partikel umfasst.

## Revendications

1. Procédé de détermination dans un échantillon d'une quantité totale de 25-hydroxy vitamine D₂ et 25-hydroxy vitamine D₃, le procédé comprenant les étapes consistant à :
(a) fournir en combinaison dans un milieu d'essai :
(i) un échantillon soupçonné de contenir de la 25-hydroxy vitamine D₂ et de la 25-hydroxy vitamine D₃,
(ii) des réactifs pour effectuer un essai pour la 25-hydroxy vitamine D₂ et la 25-hydroxy vitamine D₃, où les réactifs comprennent un anticorps d'essai capable de se lier à la 25-hydroxy vitamine D₂ et à la 25-hydroxy vitamine D₃ pour former des complexes et un élément d'un signal produisant un système qui produit un signal par rapport à la quantité des complexes 25-hydroxy vitamine D₂ et 25-hydroxy vitamine D₃, et
(iii) un anticorps non-essai qui a une plus grande affinité de liaison pour la 25-hydroxy vitamine D₂, où une quantité de l'anticorps non-essai est suffisante pour obtenir un ajustement de la contribution de la 25-hydroxy vitamine D₂ à la quantité de signal, où la quantité de l'anticorps non-essai étant de 10 µg/mL à 75 µg/mL ;
(b) incuber le milieu d'essai dans des conditions permettant la formation des complexes,
(c) déterminer la quantité de signal provenant des complexes et relier la quantité de signal à la quantité totale de 25-hydroxy vitamine D₂ et 25-hydroxy vitamine D₃ dans l'échantillon.

2. Procédé selon la revendication 1, dans lequel l'élément du système de production de signal est un analogue d'analyte de vitamine D qui comprend un marqueur dans lequel l'analogue de vitamine D est capable de se lier à l'anticorps d'essai.

3. Procédé selon la revendication 1, dans lequel les réactifs pour effectuer l'essai comprennent en outre une particule.

4. Procédé selon la revendication 1, dans lequel l'élément du système de production de signal comprend un réactif photosensibilisateur et les réactifs d'essai comprennent en outre une particule chimiluminescente, où le réactif photosensibilisateur comprend de préférence une particule.
